# EUROPEAN PATENT SPECIFICATION

(11) **EP 1 622 647 B1**
(45) Date of publication and mention of the grant of the patent: **25.11.2009**
(21) Application number: 04751990.5
(22) Date of filing: 12.05.2004
(51) Int. Cl.: A61K 47/48, A61K 49/22

(54) **VITRONECTIN RECEPTOR ANTAGONIST COMPOUNDS AND THEIR USE IN THE PREPARATION OF RADIOPHARMACEUTICALS**
VITRONECTIN REZEPTORANTAGONISTEN UND IHRE VERWENDUNG ALS RADIOPHARMAZEUTIKA
COMPOSES DE L ANTAGONISTE DU RECEPTEUR DE LA VITRONECTINE ET LEUR UTILISATION DANS LA PREPARATION DE PRODUITS RADIO PHARMACEUTIQUES

(30) Priority: 12.05.2003 US 469728 P; 11.05.2004 US 842862
(43) Date of publication of application: 08.02.2006
(73) Proprietor: Lantheus Medical Imaging, Inc., North Billerica, MA 01862 (US)
(72) Inventor: HARRIS, Thomas, D., Samel, NJ 03079 (US)
(74) Representative: Reitstötter - Kinzebach
(86) International application number: PCT/US2004/014846
(87) International publication number: WO 2004/100996

(56) References cited:
- WO-A-00/35488
- WO-A-00/35492
- WO-A-00/35887
- WO-A-02/04030
- WO-A-99/58162

## Description

### FIELD OF THE INVENTION

The present invention relates to novel compounds and their use in linking targeting moieties to imageable moieties for diagnosing and treating certain disorders in a patient

### BACKGROUND OF THE INVENTION

Disorders relating to undesirable angiogenesis include tumor growth and metastasis, inflammation, bone degradation, rheumatoid arthritis, restenosis, atherosclerosis, cardiac ischemia, and myocardial reperfusion injury. As angiogenesis depends on the agency of αvβ3 integrin (or "vitronectin receptor"), vitronectin receptor targeting moieties have been developed for therapeutic and diagnostic pharmaceuticals. *See, e*.*g*., U.S. Patent Nos. 6,548,663, 6,524,553, 6,511,649, 6,511,648, and 6,322,770.

Such therapeutic and diagnostic pharmaceuticals may include contrast agents, where one or more imageable moieties are linked to one or more of the targeting moieties, thus developing a highly specific means to detect a disorder. Typical imageable moieties are those that can be detected by X-ray CT imaging, WRI, or ultrasound. For instance, imageable moieties comprising one or more X-ray absorbing or "heavy" atoms of atomic number 20 or greater are useful as X-ray contrast agents. Imageable moieties comprising a paramagnetic metal ion are useful as magnetic resonance imaging contrast agents. Imageable moieties comprising a microbubble of a biocompatible gas, a liquid carrier, and a surfactant microsphere, are useful as ultrasound contrast agents.

If a small molecule is used to link the targeting moiety to the imageable moiety, the small molecule ("linking group") can serve a concurrent role as a pharmacokinetic modifier tor the resulting pharmaceutical.

It is an important goal to find ways of efficiently linking a targeting moiety and an imageable moiety. Thus, what is needed is a compound and method that can readily link to a variety of targeting moieties or imageable moieties. The present invention is directed to these, as well as other important ends.
EP 1 442 751 describes contrast agents comprising a vector moiety having affinity for an angiogenesis-related endothelial cell receptor, a linker moiety or a bond, and a detectable moiety. The vector may be an angiogenesis inhibitor.

Such therapeutic and diagnostic pharmaceuticals may include contrast agents, where one or more imageable moieties are linked to one or more of the targeting moieties, thus developing a highly specific means to detect a disorder. Typical imageable moieties are those that can be detected by X-ray CT imaging, MRI, or ultrasound For instance, imageable moieties comprising one or more X-ray absorbing or "heavy" atoms of atomic number 20 or greater are useful as X-ray contrast agents, Imageable moieties comprising a paramagnetic metal ion are useful as magnetic resonance imaging contrast agents. Imageable moieties comprising a microbubble of a biocompatible gas, a liquid carrier, and a surfactant microsphere, are useful as ultrasound contrast agents.

If a small molecule is used to link the targeting moiety to the imageable moiety, the small molecule ("linking group") can serve a concurrent role as a pharmacokinetic modifier for the resulting pharmaceutical.

It is an important goal to find ways of efficiently linking a targeting moiety and an imageable moiety. Thus, what is needed is a compound and method that can readily link to a variety of targeting moieties or imageable moieties. The present invention is directed to these, as well as other important ends.

EP 1 442 751 describes contrast agents comprising a vector moiety having affinity for an angiogenesis-related endothelial cell receptor, a linker moiety or a bond, and a detectable moiety. The vector may be an angiogenesis inhibitor.

### SUMMARY OF THE INVENTION

The present invention is directed, in part, to a compound comprising a thiol derivatized targeting moiety. The targeting moiety may advantageously bind to a receptor which is upregulated during angiogenesis.

In one embodiment of the present invention, there is provided a compound of the formula (V): 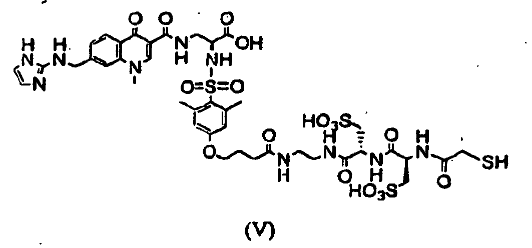 or a pharmaceutically acceptable salt thereof.

In another embodiment, the present invention provides a method of preparing the compound of formula (V), as will be described.

In another embodiment the present invention provides a method of preparing a contrast agent, comprising contacting the compound of formula (V) with a maleimide derivatized imageable moiety.

In another embodiment, the present invention providers a method of preparing a contrast agent, comprising contacting the compound of formula (V) with a α-haloacetyl derivatized imageable moiety.

These and other aspects of the invention will become more apparent from the present description and claims.

### DETAILED DESCRIPTION OF ILLUSTRATIVE EMBODIMENTS

The present invention is directed to a compound comprising a thiol derivatized targeting moiety. The targeting moiety preferably binds to u receptor which is upregulated during angiogenesis. One such receptor is the integrin αvβ3 receptor, as disclosed in U.S. Patent No. 6,511,648, the disclosure of which is hereby incorporated by reference, in its entirety.
The thiol derivatized targeting moiety is a compound of the formula (I):

(Q)_{d}-Ln-CR'(-(CR"₂)ₙSO₃H)-NH-C(=O)-Y-SH (I)

wherein Q is a targeting moiety that binds to a receptor that is upregulated during angiogenesis;
Ln is a linking group/pharmacokinetic modifier;
and d, R', R", n, and Y have the meanings that correspond to the compound of formula (V).

### Targeting moieties

The targeting moiety is a quinolone nonpeptide. Quinolone nonpeptides for use as targeting moieties are set forth in U.S. Patent Nos. 6,524,553, 6,511,649, and 6,511,648.

### Receptors

As noted above, the receptor is one associated with any protein, typically over-expressed protein, that may be expressed concurrent with a disorder in a patient. In a preferred embodiment, the receptor is the integrin αvβ3 or αvβ5.

### Linking groups/Pharmacokinetic Modifiers

As set forth in formula (I), Ln serves to link the targeting moiety Q to the remainder of the molecule, and is also preferably a pharmacokinetic modifier. Generally speaking, a pharmacokinetic modifier is a compound that serves to direct the biodistribution of an injected pharmaceutical, apart from the interaction of the targeting moiety with the receptor. Pharmacokinetic modifiers can be used, for example, to enhance or decrease hydrophilicity and rate of blood clearance, as well as to direct the route of elimination of the pharmaceuticals. Preferred pharmacokinetic modifiers are those that result in moderate to fast blood clearance and enhanced renal excretion. A wide variety of functional groups can serve as pharmacokinetic modifiers, including, hut not limited to, carbohydrates, polyalkylene glycols, peptides or other polyamino acids, and cyclodextrins.

Another embodiment of the present invention includes a method of preparing the compound of formula (I). In preferred form, the method comprises contacting a compound of the formula (VI):

(Q)_{d}-Ln-CR'(-(CR"₂)ₙSO₃H)-NH₂ (VI)

wherein, Q, d, Ln, R', R", and n, are as set forth above; with a compound having the formula (VII):

The embodiment targeting moiety is a quinolone nonpeptide. Quinolone nonpeptides for use as targeting moieties are set forth in U.S. Patent Nos. 6,524,553, 6,511,649, and 6,511,648.

### Receptors

As noted above, the receptor is one associated with any protein, typically over-expressed protein, that may be expressed concurrent with a disorder in a patient. In a preferred embodiment, the receptor is the integrin αvβ3 or αvβ5.

### Linking groups/Pharmacokinetic Modifiers

As set forth in formula (I), Ln serves to link the targeting moiety Q to the remainder of the molecule, and is also preferably a pharmacokinetic modifier. Generally speaking, a pharmacokinetic modifier is a compound that serves to direct the biodistribution of an injected pharmaceutical, apart from the interaction of the targeting moiety with the receptor. Pharmacokinetic modifiers can be used, for example, to enhance or decrease hydrophilicity and rate of blood clearance, as well as to direct the route of elimination of the pharmaceuticals. Preferred pharmacokinetic modifiers are those that result in moderate to fast blood clearance and enhanced renal excretion. A wide variety of functional groups can serve as pharmacokinetic modifiers, including, hut not limited to, carbohydrates, polyalkylene glycols, peptides or other polyamino acids, and cyclodextrins.

Another embodiment of the present invention includes a method of preparing the compound of formula (I). In preferred form, the method comprises contacting a compound of the formula (VI):

(Q)_{d}-Ln-CR'(-(CR"₂)ₙSO₃H)-NH₂ (VI)

wherein, Q, d, Ln, R', R", and n, are as set forth above; with a compound having the formula (VII):

HOOC-Y-SH (VII)

wherein, Y is as set forth above.

Methods of amide bond formation will be familiar to those skilled in the art of organic chemistry and peptide synthesis. Preferred methods include, for example, the activation of a carboxylic acid with a uronium or phosphonium salt reagent such as HBTU or PyBOP. Alternatively, carbodiimides, mixed anhydrides, symmetrical anhydrides, and EEDQ may be used for activation, as well as acid chlorides and acid fluorides.

Preferred reaction solvents include, for example, DMF, NMP, DMSO, THF, EtOAc, DCM, and chloroform. The reaction solution may be kept neutral or basic by the addition of an amine such as triethylamine or DIEA. Reactions may be carried out at ambient temperatures and protected from oxygen and water with a nitrogen atmosphere. The reactions may be carried out in solution, or with the peptide attached to a support.

Temporary protecting groups may be used to prevent other reactive functionality, such as amines, thiols, alcohols, phenols, and carboxylic acids, from participating in the reaction. Preferred amine protecting groups include, for example, t-butoxycarbonyl and trityl (removed under mild acidic conditions), Fmoc (removed by the use of secondary amines such as piperidine), and benzyloxycarbonyl (removed by strong acid or by catalytic hydrogenolysis). The trityl group may also used for the protection of thiols, phenols, and alcohols. Preferred carboxylic acid protecting groups include, for example, t-Butyl ester (removed by mild acid), benzyl ester (usually removed by catalytic hydrogenolysis), and alkyl esters such as methyl or ethyl (usually removed by mild base). All protecting groups may be removed at the conclusion of synthesis using the conditions described above for the individual protecting groups, and the final product may be purified by techniques which would be readily apparent to one of ordinary skill in the art, once armed with the present disclosure.

### Contrast agents

Another embodiment of the present invention includes a method of preparing a contrast agent. The method comprises contacting the compound of formula (I) with a maleimide derivatized imageable moiety.

As an example of such a method, a compound of formula (VIII) may be reacted with a imageable moiety comprising a maleimide group having a formula (IX) to make thioether having a formula (X): 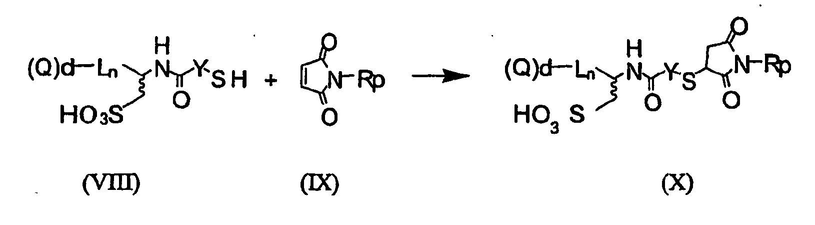 In this embodiment, Q, d, Ln, and Y are as set forth above. Rp is selected from the group of known imageable moieties for imaging.

The maleimide group reacts preferentially with thiols by a Michael addition reaction. It is commonly used for the derivatization of peptides and proteins, and methods of its use will be familiar to those skilled in the art of organic and protein chemistry. When all reagents are water soluble, the reaction may be conveniently carried out in an aqueous buffered solution at temperatures between ambient and 0 °C.

Preferred buffers include, for example, phosphate, succinate, borate, and acetate buffers. The reaction pH may typically be kept below about 8.0 to minimize reaction of the maleimide with amines. Organic co-solvents such as, for example, MeOH, ACN, or dioxane may be used when one or more reagents have limited water solubility. The use of organic solvents under anhydrous conditions is also possible.

When one of the reactants has multiple maleimide groups, as is common with proteins, the product of the conjugation reaction may contain unreacted maleimide groups. These unreacted groups may be blocked by the addition of a thiol such as methanethiol. Purification methods are determined by the nature of the conjugation product, and can include well known techniques, such as adsorption chromatography, size-exclusion chromatography, dialysis, electrophoresis, filtration, or centrifugation.

Imageable moieties include those which are well known to those skilled in the art, such as those known to be detected by X-ray CT imaging, MRI, or ultrasound.

X-ray contrast agents of the present invention may be comprised of one or more vitronectin receptor targeting moieties attached to one or more X-ray absorbing or "heavy" atoms of atomic number 20 or greater, further comprising an optional linking moiety, Ln, between the targeting moieties and the X-ray absorbing atoms. A frequently used heavy atom in X-ray contrast agents is iodine. Recently, X-ray contrast agents comprised of metal chelates (Wallace, R., U.S. Pat No. 5,417,959) and polychelates comprised of a plurality of metal ions (Love, D., U.S. Pat. No. 5,679,810) have been disclosed. More recently, multinuclear cluster complexes have been disclosed as X-ray contrast agents (U.S. Pat. No. 5,804,161, PCT WO91/14460, and PCT WO 92/17215). The disclosures of each of the foregoing documents are hereby incorporated herein by reference in their entireties.

MRI contrast agents of the present invention may be comprised of one or more vitronectin receptor targeting moieties attached to one or more paramagnetic metal ions, further comprising an optional linking moiety, Ln, between the targeting moieties and the paramagnetic metal ions. The paramagnetic metal ions may be present in the form of metal complexes or metal oxide particles. U.S. Pat. Nos. 5,412,148, and 5,760,191, describe examples of chelators for paramagnetic metal ions for use in MRI contrast agents. U.S. Pat. No. 5,801,228, U.S. Pat. No. 5,567,411, and U.S. Pat. No. 5,281,704, describe examples of polychelants useful for complexing more than one paramagnetic metal ion for use in MRI contrast agents. U.S. Pat. No. 5,520,904, describes particulate compositions comprised of paramagnetic metal ions for use as MRI contrast agents. The disclosures of each of the foregoing documents are hereby incorporated herein by reference in their entireties.

The ultrasound contrast agents of the present invention may comprise a plurality of vitronectin receptor targeting moieties attached to or incorporated into a microbubble of a biocompatible gas, a liquid carrier, and a surfactant microsphere, further comprising an optional linking moiety, Ln, between the targeting moieties and the microbubble. In this context, the term "liquid carrier" means aqueous solution and the term "surfactant" means any amphiphilic material which may produce a reduction in interfacial tension in a solution. A list of suitable surfactants for forming surfactant microspheres is disclosed, for example, in EP0727225A2, the disclosure of which is hereby incorporated herein by reference in its entirety. The term "surfactant microsphere" includes microspheres, nanospheres, liposomes, vesicles and the like. The biocompatible gas can be any physiologically accepted gas, including, for example, air, or a fluorocarbon, such as a C₃-C₅ perfluoroalkane, which provides the difference in echogenicity and thus the contrast in ultrasound imaging. The gas may be encapsulated, contained, or otherwise constrained in or by the microsphere to which is attached the biodirecting group, optionally via a linking group. The attachment can be covalent, ionic or by van der Waals forces. Specific examples of such contrast agents include, for example, lipid encapsulated perfluorocarbons with a plurality of tumor neovasculature receptor binding peptides, polypeptides or peptidomimetics. Examples of gas filled imageable moieties include those found in U.S. Patent Application Serial No. 09/931,317, filed August 16,2001, and U.S. Patent Nos. 5,088,499, 5,547,656, 5,228,446, 5,585,112, and 5,846,517, the disclosures of which are hereby incorporated herein by reference in their entireties.

Another embodiment of the present invention includes method of preparing a contrast agent. The method comprises contacting the compound of formula (I) with a α-haloacetyl derivatized imageable moiety.

### Uses

The contrast agents of the present invention may be used in a method of imaging, including methods of imaging an angiogenic event in a patient comprising administering the contrast agent to the patient by injection, infusion, or any other known method, and imaging the area of the patient wherein the angiogenic event is located. The useful dosage to be administered and the particular mode of administration will vary depending upon such factors as age, weight, and particular region to be treated, as well as the particular contrast agent used, the diagnostic use contemplated, and the form of the formulation, for example, suspension, emulsion, microsphere, liposome, or the like, as will be readily apparent to those skilled in the art. Typically, dosage is administered at lower levels and increased until the desirable diagnostic effect is achieved. In one embodiment, the above-described contrast agents may be administered by intravenous injection, usually in saline solution, at a dose of about 0.1 to about 100 mCi per 70 kg body weight (and all combinations and subcombinations of dosage ranges and specific dosages therein), or preferably at a dose of about 0.5 to about 50 mCi. Imaging is performed using techniques well known to the ordinarily skilled artisan.

For use as X-ray contrast agents, the compositions of the present invention preferably have a heavy atom concentration from about 1 mM to about 5 M, preferably about 0.1 M to about 2 M. Dosages, administered by intravenous injection, will typically range from about 0.5 mmol/kg to about 1.5 mmol/kg (and all combinations and subcombinations of dosage ranges and specific dosages therein), preferably about 0.8 mmol/kg to about 1.2 mmol/kg.

For use as MRI contrast agents, the compositions of the present invention may be used in a similar manner as other MRI agents as described in U.S. Patent No. 5,155,215; U.S. Patent No. 5,087,440; Margerstadt et al., Magn. Reson. Med., 1986, 3, 808; Runge et al., Radiology, 1988, 166, 835; and Bousquet et al., Radiology, 1988, 166, 693. The disclosures of each of the foregoing documents are hereby incorporated herein by reference in their entireties. Generally, sterile aqueous solutions of the contrast agents may be administered to a patient intravenously in dosages ranging from about 0.01 to about 1.0 mmoles per kg body weight (and all combinations and subcombinations of dosage ranges and specific dosages therein).

The ultrasound contrast agents of the present invention may be administered by intravenous injection in an amount from about 10 to about 30 µL (and all combinations and subcombinations of dosage ranges and specific dosages therein) of the echogenic gas per kg body weight or by infusion at a rate of approximately 3 µL/kg/min.

Buffers useful in the preparation of contrast agents and kits include, for example, phosphate, citrate, sulfosalicylate, and acetate buffers. A more complete list can be found in the United States Pharmacopoeia, the disclosure of which is hereby incorporated herein by reference, in its entirety.

Lyophilization aids useful in the preparation of contrast agents and kits include, for example, mannitol, lactose, sorbitol, dextran, FICOLL^{®} polymer, and polyvinylpyrrolidine (PVP).

Stabilization aids useful in the preparation of contrast agents and kits include, for example, ascorbic acid, cysteine, monothioglycerol, sodium bisulfite, sodium metabisulfite, gentisic acid, and inositol.

Solubilization aids useful in the preparation of contrast agents and kits include, for example, ethanol, glycerin, polyethylene glycol, propylene glycol, polyoxyethylene sorbitan monooleate, sorbitan monoloeate, polysorbates, poly(oxyethylene)-poly(oxypropylene)-poly(oxyethylene) block copolymers ("Pluronics") and lecithin. Preferred solubilizing aids are polyethylene glycol and Pluronics.

Bacteriostats useful in the preparation of contrast agents and kits include, for example, benzyl alcohol, benzalkonium chloride, chlorbutanol, and methyl, propyl, or butyl paraben.

A component in a diagnostic kit can also serve more than one function. For example, a reducing agent for a radionuclide can also serve as a stabilization aid, or a buffer can also serve as a transfer ligand, or a lyophilization aid can also serve as a transfer, ancillary, or co-ligand.

The compounds herein described may have asymmetric centers. Unless otherwise indicated, all chiral, diastereomeric and racemic forms are included in the present invention. Many geometric isomers of olefins, C=N double bonds, and the like can also be present in the compounds described herein, and all such stable isomers are contemplated in the present invention. It will be appreciated that compounds of the present invention contain asymmetrically substituted carbon atoms, and may be isolated in optically active or racemic forms. It is well known in the art how to prepare optically active forms, such as by resolution of racemic forms or by synthesis from optically active starting materials. Two distinct isomers (cis and trans) of the peptide bond are known to occur; both can also be present in the compounds described herein, and all such stable isomers are contemplated in the present invention. The D and L-isomers of a particular amino acid are designated herein using the conventional 3-letter abbreviation of the amino acid, as indicated by the following examples: D-Leu, or L-Leu.

When any variable occurs more than one time in any substituent or in any formula, its definition in each occurrence is independent of its definition at every other occurrence. Thus, for example, if a group, or plurality of groups, is shown to be substituted with 0-2 R⁵², then said group(s) may optionally be substituted with up to two R⁵², and R⁵² at each occurrence in each group is selected independently from the defined list of possible R⁵². Also, by way of example,
for the group -N(R⁵³)₂, each of the two R⁵³ substituents on N is independently selected from the defined list of possible R⁵³. Combinations of substituents and/or variables are permissible only if such combinations result in stable compounds. When a bond to a substituent is shown to cross the bond connecting two atoms in a ring, then such substituent may be bonded to any atom on the ring.

### Definitions

As used herein, "alkyl" is intended to include both branched and straight-chain saturated aliphatic hydrocarbon groups having the specified number of carbon atoms, examples of which include, but are not limited to, methyl, ethyl, n-propyl, i-propyl, n-butyl, i-butyl, sec-butyl, t-butyl, pentyl, hexyl, heptyl, octyl, nonyl, and decyl; cycloalkyl including saturated and partially unsaturated ring groups, including mono-, bi- or poly-cyclic ring systems, such as cyclopropyl, cyclobutyl, cyclopentyl, cyclohexyl, cycloheptyl, cyclooctyl and adamantyl; bicycloalkyl including saturated bicyclic ring groups such as [3.3.0]bicyclooctane, [4.3.0]bicyclononane, [4.4.0]bicyclodecane (decalin), [2.2.2]bicyclooctane, and so forth.

The term "alkoxy" means an alkyl-CO- group wherein alkyl is as previously described. Exemplary groups include methoxy, ethoxy, and so forth.

As used herein, the term "alkene" or "alkenyl" is intended to include hydrocarbon chains having the specified number of carbon atoms of either a straight or branched configuration and one or more unsaturated carbon-carbon bonds which may occur in any stable point along the chain, such as ethenyl, propenyl, and the like.

As used herein, the term "alkyne" or "alkynyl" is intended to include hydrocarbon chains having the specified number of carbon atoms of either a straight or branched configuration and one or more unsaturated carbon-carbon triple bonds which may occur in any stable point along the chain, such as propargyl, and the like.

As used herein, "aryl" or "aromatic residue" is intended to mean phenyl or naphthyl, which when substituted, the substitution can be at any position.

The term "aryloxy" means an aryl-CO- group wherein aryl is as previously described. Exemplary groups include phenoxy and naphthoxy.

As used herein, the term "alkaryl" means an aryl group bearing an alkyl group of 1-10 carbon atoms; the term "aralkyl" means an alkyl group of 1-10 carbon atoms bearing an aryl group; the term "arylalkaryl" means an aryl group bearing an alkyl group of 1-10 carbon atoms bearing an aryl group; and the term "heterocycloalkyl" means an alkyl group of 1-10 carbon atoms bearing a heterocycle.

"Ancillary" or "co-ligands" are ligands that may be incorporated into a radiopharmaceutical during its synthesis. They may serve to complete the coordination sphere of the radionuclide together with the chelator or radionuclide bonding unit of the reagent. For radiopharmaceuticals comprised of a binary ligand system, the radionuclide coordination sphere may be composed of one or more chelators or bonding units from one or more reagents and one or more ancillary or co-ligands, provided that there are a total of two types of ligands, chelators or bonding units. For example, a radiopharmaceutical comprised of one chelator or bonding unit from one reagent and two of the same ancillary or co-ligands and a radiopharmaceutical comprised of two chelators or bonding units from one or two reagents and one ancillary or co-ligand are both considered to be comprised of binary ligand systems. For radiopharmaceuticals comprised of a ternary ligand system, the radionuclide coordination sphere may be composed of one or more chelators or bonding units from one or more reagents and one or more of two different types of ancillary or co-ligands, provided that there are a total of three types of ligands, chelators or bonding units. For example, a radiopharmaceutical comprised of one chelator or bonding unit from one reagent and two different ancillary or co-ligands is considered to be comprised of a ternary ligand system. Ancillary or co-ligands useful in the preparation of radiopharmaceuticals and in diagnostic kits useful for the preparation of said radiopharmaceuticals may be comprised of one or more oxygen, nitrogen, carbon, sulfur, phosphorus, arsenic, selenium, and tellurium donor atoms. A ligand can be a transfer ligand in the synthesis of a radiopharmaceutical and also serve as an ancillary or co-ligand in another radiopharmaceutical. Whether a ligand is termed a transfer or ancillary or co-ligand depends on whether the ligand remains in the radionuclide coordination sphere in the radiopharmaceutical, which is determined by the coordination chemistry of the radionuclide and the chelator or bonding unit of the reagent or reagents.

"Angiogenesis" is the process of formation of new capillary blood vessels from existing vasculature. It is an important component of a variety of physiological processes including ovulation, embryonic development, wound repair, and collateral vascular generation in the myocardium. It is also central to a number of pathological conditions such as tumor growth and metastasis, diabetic retinopathy, and macular degeneration. The process begins with the activation of existing vascular endothelial cells in response to a variety of cytokines and growth factors. The activated endothelial cells secrete enzymes that degrade the basement membrane of the vessels. The endothelial cells then proliferate and migrate into the extracellular matrix first forming tubules and subsequently new blood vessels. Under normal conditions, endothelial cell proliferation is a very slow process, but it increases for a short period of time during embryogenesis, ovulation and wound healing. This temporary increase in cell turnover is governed by a combination of a number of growth stimulatory factors and growth suppressing factors. In pathological angiogenesis, this normal balance is disrupted resulting in continued increased endothelial cell proliferation. Some of the pro-angiogenic factors that have been identified include basic fibroblast growth factor (bFGF), angiogenin, TGF-alpha, TGF-beta, and vascular endothelium growth factor (VEGF), while interferon-alpha, interferon-beta and thrombospondin are examples of angiogenesis suppressors. Angiogenic factors interact with endothelial cell surface receptors such as the receptor tyrosine kinases EGFR, FGFR, PDGFR, Flk-1/KDR, Flt-1, Tek, Tie, neuropilin-1, endoglin, endosialin, and Ax1. The receptors Flk-1/KDR, neuropilin-1, and Flt-1 recognize VEGF and these interactions play key roles in VEGF-induced angiogenesis. The Tie subfamily of receptor tyrosine kinases are also expressed prominently during blood vessel formation. The proliferation and migration of endothelial cells in the extracellular matrix is mediated by interaction with a variety of cell adhesion molecules. Integrins are a diverse family of heterodimeric cell surface receptors by which endothelial cells attach to the extracellular matrix, each other and other cells. Angiogenesis induced by bFGF or TNF-alpha depend on the agency of the integrin avb3, while angiogenesis induced by VEGF depends on the integrin avb5 (Cheresh et. al., Science, 1995, 270, 1500-2). Induction of expression of the integrins a1b1 and a2b1 on the endothelial cell surface is another important mechanism by which VEGF promotes angiogenesis (Senger, et. al., Proc. Natl. Acad, Sci USA, 1997, 94, 13612-7).

A "bacteriostat" is a component that inhibits the growth of bacteria in a formulation either during its storage before use of after a diagnostic kit is used to synthesize a radiopharmaceutical.

The term "bond", as used herein, means either a single or double bond.

The term "bubbles" or "microbubbles," as used herein, refers to vesicles which are generally characterized by the presence of one or more membranes or walls surrounding an internal void that is filled with a gas or precursor thereto. Exemplary bubbles or microbubbles include, for example, liposomes, micelles and the like.

A "carbohydrate" is a polyhydroxy aldehyde, ketone, alcohol or acid, or derivatives thereof, including polymers thereof having polymeric linkages of the acetal type.

A "chelator" or "bonding unit" is the moiety or group on a reagent that binds to a metal ion through the formation of chemical bonds with one or more donor atoms. Preferred chelators of the present invention are described in U.S. Patent No. 6,511,648, the disclosure of which is hereby incorporated herein by reference in its entirety.

A "cyclodextrin" is a cyclic oligosaccharide. Examples of cyclodextrins include, but are not limited to, α-cyclodextrin, hydroxyethyl-α-cyclodextrin, hydroxypropyl-α-cyclodextrin, β-cyclodextrin, hydroxypropyl-β-cyclodextrin, carboxymethyl-β-cyclodextrin, dihydroxypropyl-β-cyclodextrin, hydroxyethyl-β-cyclodextrin, 2,6 di-O-methyl-β-cyclodextrin, sulfated-β-cyclodextin, γ-cyclodextrin, hydroxypropyl-γ-cyclodextrin, dihydroxypropyl-γ-cyclodextrin, hydroxyethyl-γ-cyclodextrin, and sulfated γ-cyclodextrin.

A "diagnostic kit" or "kit" comprises a collection of components, termed the formulation, in one or more vials which are used by the practicing end user in a clinical or pharmacy setting to synthesize diagnostic radiopharmaceuticals. The kit preferably provides all the requisite components to synthesize and use the diagnostic radiopharmaceutical except those that are commonly available to the practicing end user, such as water or saline for injection, a solution of the radionuclide, equipment for heating the kit during the synthesis of the radiopharmaceutical, if required, equipment necessary for administering the radiopharmaceutical to the patient such as syringes, shielding, imaging equipment, and the like. Therapeutic radiopharmaceuticals, X-ray contrast agent pharmaceuticals, ultrasound contrast agent pharmaceuticals and metallopharmaceuticals for magnetic resonance imaging contrast are provided to the end user in their final form in a formulation contained typically in one vial, as either a lyophilized solid or an aqueous solution. The end user typically reconstitutes the lyophilized material with water or saline and withdraws the patient dose or just withdraws the dose from the aqueous solution formulation as provided.

The term "donor atom" refers to the atom directly attached to a metal by a chemical bond.

The suffix "ene" when used with the hydrocarbons defined above, indicates that the group has two points of attachment (i.e., is a diradical). For example, a saturated aliphatic hydrocarbon group disposed between two other moieties would be referred to herein as "alkylene."

As used herein, the term "heterocycle" or "heterocyclic system" is intended to mean a stable 5- to 7- membered monocyclic or bicyclic or 7- to 10-membered bicyclic heterocyclic ring which is saturated, partially unsaturated, or unsaturated (aromatic), and which consists of carbon atoms and from 1 to 4 heteroatoms independently selected from the group consisting of N, O and S and including any bicyclic group in which any of the above-defined heterocyclic rings is fused to a benzene ring. The nitrogen and sulfur heteroatoms may optionally be oxidized. The heterocyclic ring may be attached to its pendant group at any heteroatom or carbon atom which results in a stable structure. The heterocyclic rings described herein may be substituted on carbon or on a nitrogen atom if the resulting compound is stable. If specifically noted, a nitrogen in the heterocycle may optionally be quaternized. It is preferred that when the total number of S and O atoms in the heterocycle exceeds 1, then these heteroatoms are not adjacent to one another. It is preferred that the total number of S and O atoms in the heterocycle is not more than 1. As used herein, the term "aromatic heterocyclic system" is intended to mean a stable 5-to 7- membered monocyclic or bicyclic or 7- to 10-membered bicyclic heterocyclic aromatic ring which consists of carbon atoms and from 1 to 4 heteroatoms independently selected from the group consisting ofN, O and S. It is preferred that the total number of S and O atoms in the aromatic heterocycle is not more than 1. Examples of heterocycles include, but are not limited to, 1H-indazole, 2-pyrrolidonyl, 2H,6H-1,5,2-dithiazinyl, 2H-pyrrolyl, 3H-indolyl, 4-piperidonyl, 4aH-carbazole, 4H-quinolizinyl, 6H-1,2,5-thiadiazinyl, acridinyl, azocinyl, benzimidazolyl, benzofuranyl, benzothiofuranyl, benzothiophenyl, benzoxazolyl, benzthiazolyl, benztriazolyl, benztetrazolyl, benzisoxazolyl, benzisothiazolyl, benzimidazalonyl, carbazolyl, 4a*H*-carbazolyl, carbolinyl, chromanyl, chromenyl, cinnolinyl, decahydroquinolinyl, 2*H*,6*H*-1,5,2-dithiazinyl, dihydrofuro[2,3-*b*]tetrahydrofuran, furanyl, furazanyl, imidazolidinyl, imidazolinyl, imidazolyl, 1*H*-indazolyl, indolenyl, indolinyl, indolizinyl, indolyl, isobenzofuranyl, isochromanyl, isoindazolyl, isoindolinyl, isoindolyl, isoquinolinyl, isothiazolyl, isoxazolyl, morpholinyl, naphthyridinyl, octahydroisoquinolinyl, oxadiazolyl, 1,2,3-oxadiazolyl, 1,2,4-oxadiazolyl, 1,2,5-oxadiazolyl, 1,3,4-oxadiazolyl, oxazolidinyl., oxazolyl, oxazolidinylperimidinyl, phenanthridinyl, phenanthrolinyl, phenarsazinyl, phenazinyl, phenothiazinyl, phenoxathiinyl, phenoxazinyl, phthalazinyl, piperazinyl, piperidinyl, pteridinyl, piperidonyl, 4-piperidonyl, pteridinyl, purinyl, pyranyl, pyrazinyl, pyrazolidinyl, pyrazolinyl, pyrazolyl, pyridazinyl, pyridooxazole, pyridoimidazole, pyridothiazole, pyridinyl, pyridyl, pyrimidinyl, pyrrolidinyl, pyrrolinyl, pyrrolyl, quinazolinyl, quinolinyl, 4*H*-quinolizinyl, quinoxalinyl, quinuclidinyl, carbolinyl, tetrahydrofuranyl, tetrahydroisoquinolinyl, tetrahydroquinolinyl, 6*H*-1,2,5-thiadiazinyl, 1,2,3-thiadiazolyl, 1,2,4-thiadiazolyl, 1,2,5-thiadiazolyl, 1,3,4-thiadiazolyl, thianthrenyl, thiazolyl, thienyl, thienothiazolyl, thienooxazolyl, thienoimidazolyl, thiophenyl, triazinyl, 1,2,3-triazolyl, 1,2,4-triazolyl, 1,2,5-triazolyl, 1,3,4-triazolyl, xanthenyl. Preferred heterocycles include, but are not limited to, pyridinyl, furanyl, thienyl, pyrrolyl, pyrazolyl, imidazolyl, indolyl, benzimidazolyl, 1*H*-indazolyl, oxazolidinyl, benzotriazolyl, benzisoxazolyl, oxindolyl, benzoxazolinyl, or isatinoyl. Also included are fused ring and spiro compounds containing, for example, the above heterocycles.

As used herein, the term "lipid" refers to a synthetic or naturally-occurring amphipathic compound which comprises a hydrophilic component and a hydrophobic component. Lipids include, for example, fatty acids, neutral fats, phosphatides, glycolipids, aliphatic alcohols and waxes, terpenes and steroids. Exemplary compositions which comprise a lipid compound include suspensions, emulsions and vesicular compositions.

"Liposome" refers to a generally spherical cluster or aggregate of amphipathic compounds, including lipid compounds, typically in the form of one or more concentric layers, for example, bilayers. They may also be referred to herein as lipid vesicles.

A "lyophilization aid" is a component that has favorable physical properties for lyophilization, such as the glass transition temperature, and is generally added to the formulation to improve the physical properties of the combination of all the components of the formulation for lyophilization.

"Metallopharmaceutical" means a pharmaceutical comprising a metal. The metal is the cause of the imageable signal in diagnostic applications and the source of the cytotoxic radiation in radiotherapeutic applications. Radiopharmaceuticals are metallopharmaceuticals in which the metal is a radioisotope.

The term "nonpeptide" means preferably less than three amide bonds in the backbone core of the targeting moiety or preferably less than three amino acids or amino acid mimetics in the targeting moiety.

The phrase "pharmaceutically acceptable" is employed herein to refer to those compounds, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

As used herein, "pharmaceutically acceptable salts" refer to derivatives of the disclosed compounds modified by making acid or base salts. Examples of pharmaceutically acceptable salts include, but are not limited to, mineral or organic acid salts of basic residues such as amines; alkali or organic salts of acidic residues such as carboxylic acids; and the like. The pharmaceutically acceptable salts include the conventional non-toxic salts or the quaternary ammonium salts of the parent compound formed, for example, from non-toxic inorganic or organic acids. For example, such conventional non-toxic salts include those derived from inorganic acids such as hydrochloric, hydrobromic, sulfuric, sulfamic, phosphoric, nitric and the like; and the salts prepared from organic acids such as acetic, propionic, succinic, glycolic, stearic, lactic, tartaric, citric, ascorbic, pamoic, maleic, hydroxymaleic, phenylacetic, glutamic, benzoic, salicylic, sulfanilic, 2-acetoxybenzoic, fumaric, toluenesulfonic, methanesulfonic, ethane disulfonic, oxalic, isethionic, and the like. The pharmaceutically acceptable salts of the present invention can be synthesized from the parent compound which contains a basic or acidic moiety by conventional chemical methods. Generally, such salts can be prepared by reacting the free acid or base forms of these compounds with a stoichiometric amount of the appropriate base or acid in water or in an organic solvent, or in a mixture of the two; generally, nonaqueous media like ether, ethyl acetate, ethanol, isopropanol, or acetonitrile are preferred. Lists of suitable salts are found in Remington's Pharmaceutical Sciences, 17th ed., Mack Publishing Company, Easton, PA, 1985, p. 1418, the disclosure of which is hereby incorporated by reference.

A "polyalkylene glycol" is a polyethylene glycol, polypropylene glycol, polybutylene glycol, or similar glycol having a molecular weight of less than about 5000, terminating in either a hydroxy or alkyl ether moiety.

As used herein, the term "polycarboxyalkyl" means an alkyl group having from about two and about 100 carbon atoms and a plurality of carboxyl substituents; and the term "polyazaalkyl" means a linear or branched alkyl group having from about two and about 100 carbon atoms, interrupted by or substituted with a plurality of amine groups.

By "reagent" is meant a compound of this invention capable of direct transformation into a metallopharmaceutical of this invention. Reagents may be utilized directly for the preparation of the metallopharmaceuticals of this invention or may be a component in a kit of this invention.

A "reducing agent" is a compound that reacts with a radionuclide, which is typically obtained as a relatively unreactive, high oxidation state compound, to lower its oxidation state by transferring electron(s) to the radionuclide, thereby making it more reactive. Reducing agents useful in the preparation of radiopharmaceuticals and in diagnostic kits useful for the preparation of said radiopharmaceuticals include, for example, stannous chloride, stannous fluoride, formamidine sulfinic acid, ascorbic acid, cysteine, phosphines, and cuprous or ferrous salts. Other reducing agents are described, for example, in Brodack et. al., PCT Application 94/22496, the disclosure of which is incorporated herein by reference in its entirety.

The term "salt", as used herein, is used as defined in the CRC Handbook of Chemistry and Physics, 65th Edition, CRC Press, Boca Raton, Fla, 1984, as any substance which yields ions, other than hydrogen or hydroxyl ions.

A "stabilization aid" is a component that is typically added to the metallopharmaceutical or to the diagnostic kit either to stabilize the metallopharmaceutical or to prolong the shelf-life of the kit before it must be used. Stabilization aids can be antioxidants, reducing agents or radical scavengers and can provide improved stability by reacting preferentially with species that degrade other components or the metallopharmaceutical.

By "stable compound" or "stable structure" is meant herein a compound that is sufficiently robust to survive isolation to a useful degree of purity from a reaction mixture, and formulation into an efficacious pharmaceutical agent.

A "solubilization aid" is a component that improves the solubility of one or more other components in the medium required for the formulation.

The term "substituted", as used herein, means that one or more hydrogens on the designated atom or group is replaced with a selection from the indicated group, provided that the designated atom's or group's normal valency is not exceeded, and that the substitution results in a stable compound. When a substituent is keto (i.e., =O), then 2 hydrogens on the atom are replaced.

A "transfer ligand" is a ligand that forms an intermediate complex with a metal ion that is stable enough to prevent unwanted side-reactions but labile enough to be converted to a metallopharmaceutical. The formation of the intermediate complex is kinetically favored while the formation of the metallopharmaceutical is thermodynamically favored. Transfer ligands useful in the preparation of metallopharmaceuticals and in diagnostic kits useful for the preparation of diagnostic radiopharmaceuticals include, for example, gluconate, glucoheptonate, mannitol, glucarate, N,N,N,N'-ethylenediaminetetraacetic acid, pyrophosphate and methylenediphosphonate. In general, transfer ligands are comprised of oxygen or nitrogen donor atoms.

As used herein, the term "vesicle" refers to a spherical entity which is characterized by the presence of an internal void. Preferred vesicles are formulated from lipids, including the various lipids described herein. In any given vesicle, the lipids may be in the form of a monolayer or bilayer, and the mono- or bilayer lipids may be used to form one of more mono- or bilayers. In the case of more than one mono- or bilayer, the mono- or bilayers are generally concentric. The lipid vesicles described herein include such entities commonly referred to as liposomes, micelles, bubbles, microbubbles, microspheres and the like. Thus, the lipids may be used to form a unilamellar vesicle (comprised of one monolayer or bilayer), an oligolamellar vesicle (comprised of about two or about three monolayers or bilayers) or a multilamellar vesicle (comprised of more than about three monolayers or bilayers). The internal void of the vesicles may be filled with a liquid, including, for example, an aqueous liquid, a gas, a gaseous precursor, and/or a solid or solute material, including, for example, a bioactive agent, as desired.

As used herein, the term "vesicular composition" refers to a composition which is formulate from lipids and which comprises vesicles.

As used herein, the term "vesicle formulation" refers to a composition which comprises vesicles and a bioactive agent.

The following abbreviations are used herein:
- Acm: acetamidomethyl
- b-Ala, beta-Ala or bAla: 3-aminopropionic acid
- ATA: 2-aminothiazole-5-acetic acid or 2-aminothiazole-5-acetyl group
- Boc: t-butyloxycarbonyl
- CBZ, Cbz or Z: Carbobenzyloxy
- Cit: citrulline
- Dap: 2,3-diaminopropionic acid
- DCC: dicyclohexylcarbodiimide
- DIEA: diisopropylethylamine
- DMAP: 4-dimethylaminopyridine
- EOE: ethoxyethyl
- HBTU: 2-(1H-Benzotriazol-1-yl)-1,1,3,3- tetramethyluronium hexafluorophosphate
- hynic: boc-hydrazinonicotinyl group or 2-[[[5-[carbonyl]-2-pyridinyl] hydrazono]methyl]-benzenesulfonic acid,
- NMeArg or MeArg: a-N-methyl arginine
- NMeAsp: a-N-methyl aspartic acid
- NMM: N-methylmorpholine
- OcHex: O-cyclohexyl
- OBzl: O-benzyl
- oSu: O-succinimidyl
- TBTU: 2-(1H-Benzotriazol-1-yl)-1,1,3,3-tetramethyluronium tetrafluoroborate
- THF: tetrahydrofuranyl
- THP: tetrahydropyranyl
- Tos: tosyl
- Tr or Trt: trityl

The present invention is further described in the following examples.

### EXAMPLES

### Example 1

### Synthesis of 4-(4-(((2-((tert-Butoxy))carbonylamino)-1-(methoxycarbonyl)ethyl)amino)sulfonyl)-3,5-(dimethylphenoxy)butanoic Acid

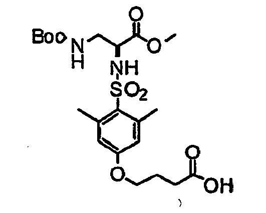

### Part A - Ethyl 4-(3,5-Dimethylphenoxy)butanoate

Sodium metal (17.12 g, 0.744 mol) was added to anhydrous EtOH (350 mL) and stirred until dissolved. 3,5-Dimethylphenol was added and the solution was stirred 15 min at ambient temperatures. Ethyl 4-bromoacetate (58.7 mL, 0.41 mol) was added and the solution was stirred at ambient temperatures under a nitrogen atmosphere for 28 h. The EtOH was removed under vacuum and the oily solid was partitioned between water (1 L) and EtOAc (500 mL). The aqueous layer was extracted with additional EtOAc (500 mL). The combined EtOAc extracts were washed consecutively with saturated NaHCO₃ (300 mL) and saturated NaCl (300 mL), dried (MgSO₄), and concentrated to give an amber liquid. This liquid was vacuum fractional distilled through a 15 cm Vigreux column. The main fraction was collected from 91-117 °C/6 mm Hg to gave the title compound as a colorless liquid (77.77 g, 89%). ¹H NMR (CDCl₃): 6.59 (s, 1H), 6.52 (s, 2H), 4.16 (q, J - 7.16 Hz, 2H), 3.98 (t, J = 6.14 Hz, 2H), 2.49 (t, J = 7.34 Hz, 2H), 2.28 (s, 6H), 2.11-2.07 (m, 2H), 1.26 (t, J = 7.16 Hz, 3H); Anal. calcd for C₁₄H₂O₃: C,71.16; H, 8.53, Found: C,71.35; H, 8.59.

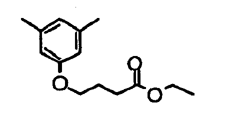

### Part B - 4-(3,5-Dimethylphenoxy)butanoic Acid

The product of part A, above (75.52 g, 0.320 mol) and KOH pellets (38.5 g, 0.584 mol) were dissolved in absolute EtOH (1.50 L) and heated at reflux for 3 h. The solution was concentrated to a colorless solid, which was taken up in water (2.0 L) and washed with ether (2 x 750 mL). The aqueous layer was adjusted to pH 1 with concd HCl (55 mL) and the resulting oily ppt was extracted into EtOAc (2 x 500 mL). The combined EtOAc extracts were washed consecutively with water (300 mL) and saturated NaCl, dried (MgSO₄), and concentrated to give a colorless solid (64.13 g). Recrystallization from hexanes (500 mL) gave the title compound as a colorless solid (59.51 g, 89%). MP: 66-68.5 °C; ¹H NMR (CDCl₃): 11.70 (bs, 1H), 6.59 (s, 1H), 6.52 (s, 2H), 3.99 (t, J = 6.06 Hz, 2H), 2.57 (t, J = 7.29 Hz, 2H), 2.28 (s, 6H), 2.12-2.08 (m, 2H); Anal. calcd for C₁₂H₁₆O₃: C, 69.21; H, 7.74, Found: C, 69.23; H, 7.40.

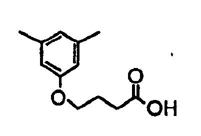

### Part C - 4-(4-(Chlorosulfonyl)-3,5-dimethyhlhenoxy)butanoic Acid

A solution of the product of Part B, above (20.8 g, 0.100 mol) in CHCl₃ (100 mL) was cooled to 0 °C and treated with chlorosulfonic acid (36 mL, 0.54 mol) dropwise and with rapid stirring while keeping the temperature of the reaction at 0 °C. The resulting gelatinous mixture was stirred an additional 10 min and poured onto an ice/water mixture (600 mL). The resulting solid ppt was collected by filtration, washed with water (3 x 75 mL), and dried under vacuum to give a colorless solid (12.52 g). MP: 114-115 °C (with decomp); ¹H NMR (CDCl₃): 13.84 (bs, 1H), 6.50 (s, 2H), 3.91 (t, J = 6.48 Hz, 2H), 2.48 (s, 6H), 2.32 (t, J = 7.32 Hz, 2H), 1.89-1.84 (m, 2H); IR (KBr cm⁻¹): 1705 (s), 1370 (s), 1175 (s); MS: m/e 305.1 [M-H].

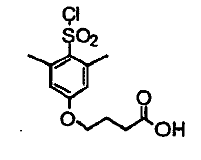

### Part D - 4-(4-(((2-((tert-Butoxy)carbonylamino)-1-(methoxycarbonyl)ethyl)amino)sulfonyl)-3,5-dimethylphenoxy)butanoic Acid

A solution of N-β-Boc-L-α β,-diaminopropionic acid methyl ester hydrochloride (568 mg, 2.10 mmol) and DIEA (0.73 mL, 4.2 mmol) in DCM (5 mL) was cooled to 0 °C and treated with a suspension of the product of Part C, above (656 mg, 2.10 mmol) in DCM (20 mL) in small portions over a 15 min period. The reaction was stirred at ambient temperatures under a nitrogen atmosphere for 18 h. The reaction was diluted with DCM (100 mL) and washed with water (3 x 75 mL). The organic phase was dried (MgSO₄), and concentrated to give crude product (698 mg), which was purified by preparative HPLC on a Vydac C-18 column (50 x 250 mm) using a 0.96%/min gradient of 18 to 58.5% ACN containing 0.1% TFA at a flow rate of 80 mL/min. The main product fraction eluting at 23.8 min was collected adjusted to pH 3, partially concentrated to remove ACN, and extracted with DCM (2 x 100 mL). The DCM extracts were dried (MgSO₄) and concentrated to give the title compound as a colorless solid (297 mg, 29%). ¹H NMR (CDCl₃): δ 6.61 (s, 2H), 5.66 (d, J = 7.2 Hz, 1H), 4.90 (s, 1H), 4.03 (bs, 2H), 3.86 (bs, 1H), 3.59 (s, 3H), 3.49 (bs, 2H), 2.62 (s, 6H), 2.58-2.51 (m, 2H), 2.18-2.07 (m, 2H), 1.41 (s, 9H); . MS: m/e 489.4 [M+H]; High Resolution MS: Calcd for C₂₁H₃₃N₂O₉S [M+Na]: 511.1726, Found: 511.1747; Anal. calcd for C₂₁H₃₂N₂O₉S: C, 51.62; H, 6.61; N, 5.74, Found: C, 51.47; H, 6.27; N, 5.48.

### Example 2

### Synthesis of (2R)-N-[(1R)-1-(N-{2-[4-(4-{[((1S)-1-(Methoxycarbonyl)-2-{[1-methyl-4-oxo-7-({[1-(triphenylmethyl)imidazol-2-yl]amino}methyl)(3-hydroquinolyl)]carbonylamino}ethyl)amino]sulfonyl}-3,5-dimethylphenoxy)butanoylamino]ethyl}carbamoyl)-2-sulfoethyl]-2-aminopropanesulfonic Acid

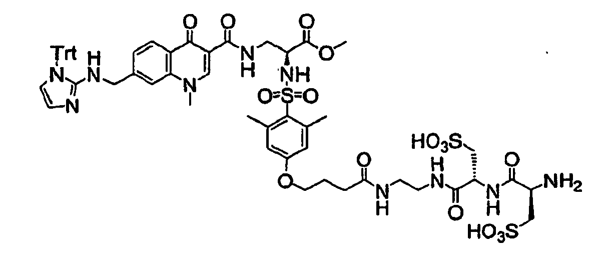

### Part A Preparation of Methyl (2S)-3-[(tert-Butoxy)carbonylamino]-2-[({2,6-dimethyl-4-[3-(N-{2-[(phenylmethoxy)carbonylamino]ethyl}carbamoyl)propoxy]-phenyl}sulfonyl)amino]propanoate

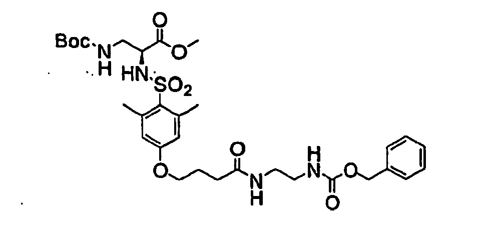

A solution of the product of Example 1, Part D (369 mg, 0.756 mmol), DIEA (0.52 mL, 3.0 mmol), and HBTU (315 mg, 0.832 mmol) in anhydrous DMF (14 mL) was stirred at ambient temperatures under nitrogen for 5 min, and treated with benzyl N-(2-aminoethyl)carbamate hydrochloride (192 mg, 0.832 mmol), and stirred an additional 1 h. The DMF was removed under vacuum, and the oily residue was taken up in EtOAc (150 mL), washed consecutively with 0.1 N HCl (40 mL), water (40 mL), and saturated NaCl (40 mL), dried (MgSO₄), and concentrated to give a colorless viscous oil. Flash chromatography on a 3 x 16 cm silica gel column (EtOAc) gave the title compound as a colorless viscous oil (450 mg, 89.6%). ¹H NMR (CDCl₃): δ 7.34-7.27 (m, 5H), 6.58 (s, 2H), 6.31 (bs, 1H), 5.86 (bs, 1H), 5.36 (bs, 1H); 5.14-5.03 (m, 3H), 3.96 (t, J = 6.0 Hz, 2H), 3.88-3.83 (m, 1H), 3.56 (s, 3H), 3.47-3.25 (m, 6H), 2.59 (s, 6H), 2.31 (t, J = 6.9 Hz, 2H), 2.05 (p, J = 6.6 Hz, 2H), 1.39 (s, 9H); ¹³C NMR (CDCl₃): δ 172.9, 170.5, 160.6, 157.3, 155.9, 141.8, 136.3, 128.5, 128.2, 128.0, 116.6, 79.9, 66.9, 55.5, 52.8, 43.1, 40.9, 40.3, 32.4, 28.2, 24.9, 23.3; MS: m/e 665.4 [M+H]; 687.3 [M+Na]; High Resolution MS: Calcd for C₃₁H₄₅N₄O₁₀S [M+H]: 665.2856, Found: 665.2883.

### Part B - Preparation of Methyl (2S)-3-Amino-2-[({2,6-dimethyl-4-[3-(N-{2-[(phenylmethoxy)carbonylamino]ethyl} carbamoyl)propoxy]phenyl}sulfonyl)amino]propanoate Trifluoroacetate Salt

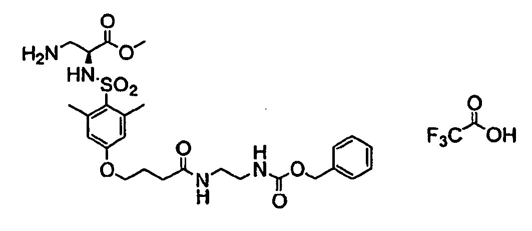

The product of Part A, above (420 mg, 0.632 mmol) was dissolved in 25/75 DCM/TFA (20 mL) and allowed to stand at ambient temperatures under nitrogen for 10 min. The solution was concentrated, and the resulting viscous oil was dissolved in 50% ACN and lyophilized to give the title compound as a colorless solid (437 mg, 102%). MS: m/e 565.3 [M+H].

### Part C - Preparation of Methyl (2S)-2-[({2,6-Dimethyl-4-[3-(N-{2-[(phenylmethoxy)carbonylamino]ethyl}carbamoyl)propoxy]phenyl}sulfonyl)amino]-3-{[1-methyl-4-oxo-7-({[1-(triphenylmethyl)imidazol-2-yl]amino}methyl)(3-hydroquinolyl)]carbonylamino}propanoate

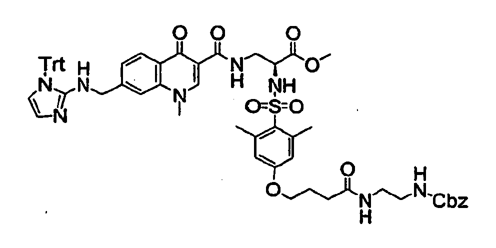

A solution of 1-methyl-4-oxo-7-(((1-(triphenylmethyl)imidazol-2-yl)amino)methyl)hydroquinoline-3-carboxylic acid (702 mg, 1.30 mmol), DIEA (0.678 mL, 3.90 mmol), and HBTU (542 mg, 1.43 mmol) in anhydrous DMF (60 mL) was stirred at ambient temperatures under nitrogen for 10 min, and treated with the product of Step B, above (881 mg, 1.30 mmol). After 75 min the DMF was removed under vacuum and the resulting oil was purified by HPLC on a Vydac C-18 column (50 x 250 mm) using a 1.24%/min gradient of 18 to 67.5% ACN containing 0.1% TFA at a flow rate of 80 mL/min. A peak eluting at 18.9 min was lyophilized to give unreacted 1-methyl-4-oxo-7-(((1-(triphenylmethyl)imidazol-2-yl)amino)methyl)hydroquinoune-3-carboxylic acid (308 mg). The main product peak eluting at 23.7 min was lyophilized to give the title compound as a colorless solid (890 mg, 63.0%). ¹H NMR (CDCl₃/D₂O): δ 8.50 (s, 1H), 8.18 (d, J = 8.3 Hz, 1H), 7.70 (s, 1H), 7.51-7.25 (m, 15H), 7.25-7.12 (m, 5H), 6.97 (s, 1H), 6.58 (d, J = 2.3 Hz, 1H), 6.34 (s, 2H), 6.32 (d, J = 8.5 Hz, 1H), 5.09 (s, 2H), 4.65 (s, 2H), 4.29-4.23 (m, 1H), 3.88 (s, 3H), 3.80-3.50 (m, 7H), 3.41-3.28 (m, 4H), 2.61 (s, 6H), 2.26-2.11 (m, 2H), 1.92-1.76 (m, 2H); MS: m/e 1087.4 [M+H]; 845.3 [M+H-Tr]; High Resolution MS: Calcd for C₆₀H₆₃N₈O₁₀S [M+H]: 1087.4388; found: 1087.440.

### Part D - Preparation of Methyl (2S)-2-{[(4-{3-[N-(2-Aminoethyl)carbamoyl]propoxy}-2,6-dimethylphenyl)sulfonyl]amino}-3-{[1-methyl-4-oxo-7-({[1-(triphenylmethyl)imidazol-2-yl]amino}methyl)(3-hydroquinolyl)]carbonylamino}propanoate

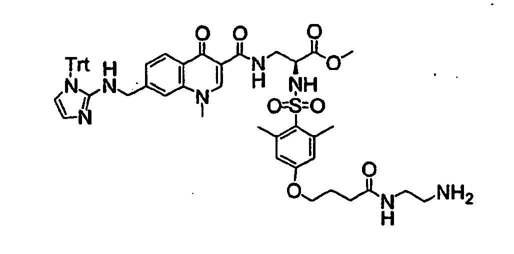

Hydrogenolysis of the product of Part C, above (468 mg, 0.431 mmol) was accomplished in MeOH (100 mL) over 10% Pd/C (95 mg) at 60 psi for 1 h. The catalyst was removed by filtration through CELITE® and the filtrate was concentrated to give the title compound as a pale amber oil (405 mg, 98.7%). MS: m/e 953.3 [M+H], 711.3 [M+H-Trityl].

### Part E - Preparation of (2R)-N-{2-[4-(4-{[((1S)-1-(Methoxycarbonyl)-2-{[1-methyl-4-oxo-7-({[1-(triphenylmethyl)imidazol-2-yl]amino}methyl)(3-hydroquinolyl)]carbonylaxnino}ethyl)amino]sulfonyl}-3,5-dimethylphenoxy)butanoylamino]ethyl}-2-[(tert-butoxy)carbonylamino]propanesulfonic Acid

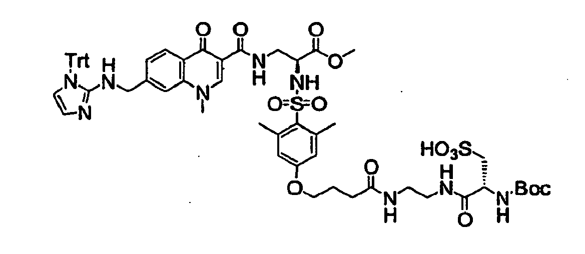

A solution of the product of Part D, above (405 mg, 0.425 mmol), the p-nitrophenyl ester of Boc-L-cysteic acid (425 mg, 1.03 mmol), and DIEA (0.435 mL, 2.55 mmol) in anhydrous DMF (20 mL) was stirred at ambient temperatures under nitrogen for 3 h. The DMF was removed under vacuum and the resulting oil was purified by HPLC on a Vydac C-18 column (50 x 250 mm) using a 1.12%/min gradient of 9 to 54% ACN containing 0.1% TFA at a flow rate of 80 mL/min. The main product peak eluting at 37.3 min was lyophilized to give the title compound as a colorless solid (410 mg, 80.2%). MS: m/e 1204.4 [M+H], 962.3 [M+H-Trt].

### Part F - Preparation of (2R)-N-{2-[4-(4-{[((1S)-1-(Methoxycarbonyl)-2-{[1-methy]4-oxo-7-({[1-(triphenylmethyl)imidazol-2-yl]amino}methyl)(3-hydroquinolyl)]carbonylamino}ethyl)amino]sulfonyl}-3,5-dimethylphenoxy)butanoylamino]ethyl}-2-aminopropanesulfonic Acid

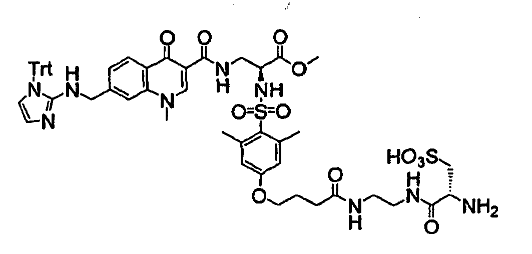

The product of Part E, above (410 mg, 0.341 mmol) was dissolved in 50/50 TFA/DCM (20 mL) and allowed to react at ambient temperatures for 10 min. The solution was concentrated and the resulting amber oil was dissolved in 50% ACN (50 mL) and lyophilized to give the title compound as a colorless solid (371 mg, 98.6%). MS: m/e 1104.4 [M+H], 862.3 [M+H-Trt]; High Resolution MS: Calcd for C₅₅H₆₂N₉O₁₂S₂ [M+H]: 1104.3959; Found: 1104.393.

### Part G - Preparation of (2R)-N-[(1R)-1-(N-{2-[4-(4-{[((1S)-1-(Methoxycarbonyl)-2-{[1-methyl-4-oxo-7-({[1-(triphenylmethyl)imidazol-2-yl]amino}methyl)(3-hydroquinolyl)]carbonylamino}ethyl)amino]sulfonyl}-3,5-dimethylphenoxy)butanoylamino]ethyl}carbamoyl)-2-sulfoethyl]-2-[(tert-butoxy)carbonylamino]propanesulfonic Acid

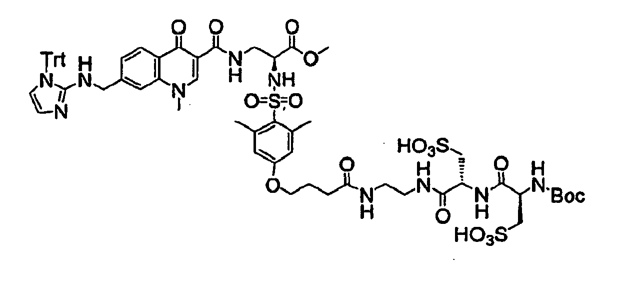

A solution of the product of Part F, above (110 mg, 0.100 mmol), the p-nitrophenyl ester of Boc-L-cysteic acid (82.4 mg, 0.200 mmol), and DIEA (0.104 mL, 0.600 mmol) in anhydrous DMF (5.0 mL) was stirred at ambient temperatures under nitrogen for 48 h. The DMF was removed under vacuum and the resulting amber oil was purified by HPLC on a Vydac C-18 column (50 x 250 mm) using a 1.12%/min gradient of 9 to 54% ACN containing 0.1% TFA at a flow rate of 80 mL/min. The main product peak eluting at 37.0 min was lyophilized to give the title compound as a colorless solid (96.0 mg, 70.9%). MS: m/e 1355.3 [M+H], 1113.3 [M-Trt+H], 1013.2 [M-Trt-Boc+H].

### Part H - Preparation of (2R)-N-[(1R)-1-(N-{2-[4-(4-{[((1S)-1-(Methoxycarbonyl)-2-{[1-methyl-4-oxo-7-({[1-(triphenylmethyl)imidazol-2-yl]amino}methyl)(3-hydroquinolyl)]carbonylamino}ethyl)amino]sulfonyl}-3,5-dimethylphenoxy)butanoylamino]ethyl}carbamoyl)-2-sulfoethyl]-2-aminopropanesulfonic Acid

The product of Part G, above (21 mg, 0.0155 mmol) was dissolved in 50/50 TFA/DCM (5.0 mL) and allowed to react at ambient temperatures for 10 min. The solution was concentrated and the residue was taken up in 50% ACN (15 mL) and lyophilized to give the title compound as a colorless solid (18.7 mg, 96.2%). MS: m/e 1255.3 [M+H], 1013.2 [M+H-Trityl]; High Resolution MS: Calcd for C₅₈H₆₇N₁₀O₁₆S₃ [M+H]: 1255.3899; Found: 1255.391.

### Example 3

### Synthesis of 2-[({4-[3-(N-{2-[(2R)-2-((2R)-2-Amino-3-sulfopropyl)-3-sulfopropyl]ethyl}-carbamoyl)propoxy]-2,6-dimethylphenyl}sulfonyl)amino](2S)-3-{[1-methyl-4-oxo-7-({[1-(triphenylmethyl)imidazol-2-yl]amino}methyl)(3-hydroquinolyl)]carbonylamino}propanoic Acid

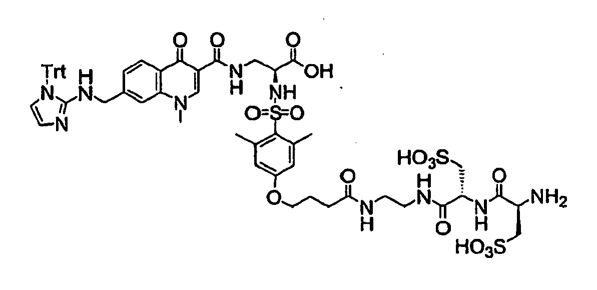

### Part A - Preparation of 2-({[4-(3-{N-[2-((2R)-2-Amino-3-sulfopropyl)ethyl]carbamoyl}propoxy)-2,6-dimethylphenyl]sulfonyl}amino)(2S)-3-{[1-methyl-4-oxo-7-({[1-(triphenylmethyl)imidazol-2-yl]amino}methyl)(3-hydroquinolyl)]carbonylamino}propanoic Acid

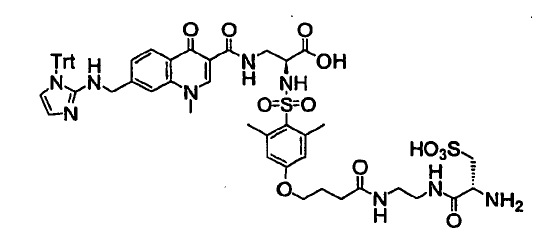

A mixture of the product of Example 2, Part F (125 mg, 0.113 mmol), peroxide-free THF (3.8 mL), water (0.57 mL), and 3 N LiOH (0.38 mL, 1.13 mmol) was stirred at ambient temperatures under nitrogen for 1 h. The mixture was adjusted to pH 1 using 1 N HCl (0.70 mL) and concentrated to dryness under vacuum. The resulting solid was purified by HPLC on a Vydac C-18 column (50 x 250 mm) using a 0.90%/min gradient of 18 to 54% ACN containing 0.1% TFA at a flow rate of 80 mL/min. The main product peak eluting at 21.0 min was lyophilized to give the title compound as a colorless solid (96.0 mg, 77.9%). MS: m/e 1090.3 [M+H], 848.2 [M+H-Trt]; High Resolution MS: Calcd for C₅₄H₆₀N₉O₁₂S₂ [M+H]: 1090.3808; Found: 1090.381.

### Part B - Preparation of 2-({[4-(3-{N-[2-((2R)-2-{(2R)-2-[(tert-Butoxy)carbonylamino]-3-sulfopropyl}-3-sulfopropyl)ethyl]carbamoyl}propoxy)-2,6-dimethylphenyl]sulfonyl}amino)-(2S)-3-{[1-methyl-4-oxo-7-({[1-(triphenylmethyl)imidazol-2-yl]amino}methyl)(3-hydroquinolyl)]carbonylamino}propanoic Acid

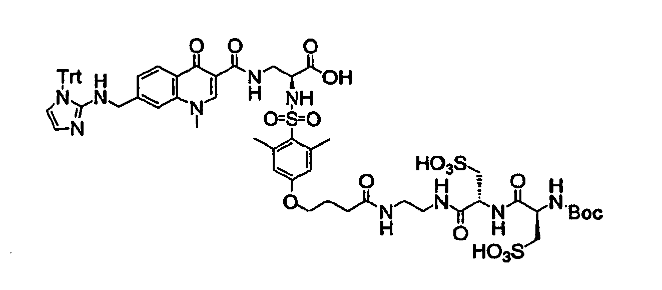

A solution of Boc-L-cysteic acid (37.0 mg, 0.128 mmol), DIEA (0.040 mL, 0.228 mmol), and PyBOP (53.0 mg, 0.102 mmol) in anhydrous DMF (1.0 mL) was stirred at ambient temperatures under nitrogen for 15 min, and added to a solution of the product of Part A, above (93.0 mg, 0.0854 mmol) and DIEA (0.045 mL, 0.256 mmol) in anhydrous DMF (3.0 mL). The resulting solution was stirred at ambient temperatures under nitrogen for 1.5 h and concentrated to a viscous amber oil. Purification by HPLC on a Vydac C-18 column (50 x 250 mm) using a 0.68%/min gradient of 18 to 45% ACN containing 0.1% TFA at a flow rate of 80 mL/min. The main product peak eluting at 36.4 min was lyophilized to give the title compound as a colorless solid (94.0 mg, 82.1%). MS: m/e 1341.2 [M+H], 1099.1 [M+H-Trt], 999.1 [M+H-Trt-Boc].

### Part C - Preparation of (2R)-N-[(1R)-1-(N-{2-[4-(4-{[((1S)-1-(Methoxycarbonyl)-2-{[1-methyl-4-oxo-7-({[1-(triphenylmethyl)imidazol-2-yl]amino}methyl)(3-hydroquinolyl)]carbonylamino}ethyl)aniino]sulfonyl}-3,5-dimethylphenoxy)butanoylamino]ethyl}carbamoyl)-2-sulfoethyl]-2-aminopropanesulfonic Acid

A solution of the product of Part B, above (90.0 mg, 0.0672 mmol) in 50/50 TFA/DCM (10.0 mL) was allowed to react at ambient temperatures under nitrogen for 10 min and concentrated under vacuum to give the intermediate amine as an amber oil. MS: m/e 1241.3 [M+H], 999.3 [M+H-Trt]; High Resolution MS: Calcd for C₅₇H₆₅N₁₀O₁₆S₃ [M+H]: 1241.3742; Found: 1241.375

### Example 4

### Synthesis of 2-{[(4-{3-[N-(2-{(2R)-2-[(2R)-2-(2-Sulfanylacetylamino)-3-sulfopropyl]-3-sulfopropyl}ethyl)carbamoyl]propoxy}-2,6-dimethylphenyl)sulfonyl]amino}(2S)-3-({7-[(imidazol-2-ylamino)methyl]-1-methyl-4-oxo(3-hydroquinolyl)}carbonylamino)propanoic Acid

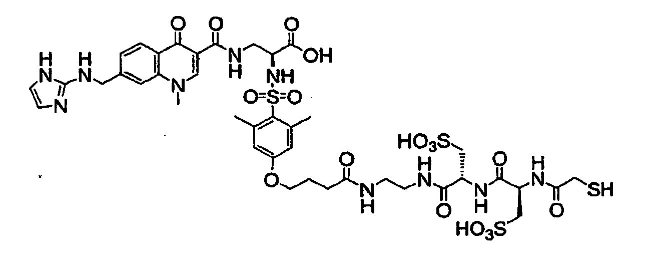

### Part A - Preparation of 2-({[4-(3-{N-[2-((2R)-2-{(2R)-3-Sulfo-2-[2-(triphenylmethylthio)-acetylamino]propyl}-3-sulfopropyl)ethyl]carbamoyl}propoxy)-2,6-dimethylphenyl]-sulfonyl}amino)(2S)-3-{[1-methyl-4-oxo-7-({[1-(triphenylmethyl)imidazol-2-yl]amino}-methyl)(3-hydroquinolyl)]carbonylamino}propanoic Acid

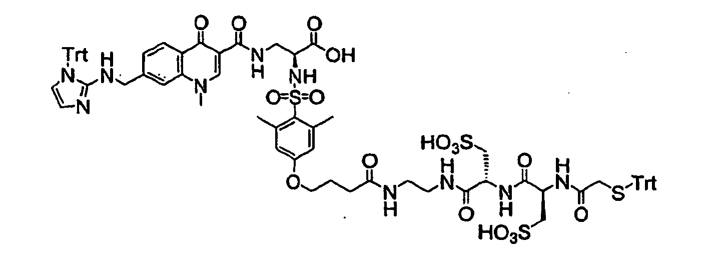

A solution of 2-((triphenylmethyl)thio)acetic acid (Brenner, D, et al. Inorg. Chem. 1984, 23, 3793-3797)(48.3 mg, 0.145 mmol) and HOAt (5.2 mg, 0.0386 mmol) in DMF (2.0 mL) was made basic with DIEA (0.080 mL) and treated with HBTU (43.8 mg, 0.116 mmol). The resulting solution was stirred at ambient temperatures under nitrogen for 15 min resulting in a pale yellow solution. In a separate flask a solution of the product of Example 3, Part C (40 mg, 0.032 mmol) in DMF (2.0 mL) was made basic with DIEA (0.022 mL). The two solutions were combined and the resulting pale yellow solution was stirred under nitrogen for 22 h. The solution was concentrated to give an amber oil, which was purified by HPLC on a Phenomenex Luna C-18 column (41.4 x 250 mm) using a 0.45%/min gradient of 31.5 to 49.5% ACN containing 0.1% TFA at a flow rate of 80 mL/min. The main product peak eluting at 36 min was lyophilized to give the title compound as a colorless solid (20.6 mg, 41.1%). MS: m/e 1558.8 [M+H] (32%), 1315.7 [M+H-Trt] (27%); High Resolution MS: Calcd for C₇₈H₇₉N₁₀O₁₇S₄ [M-2H]: 777.2214; Found: 777.2224.

### Part B - Preparation of 2-{[(4-{3-[N-(2-{(2R)-2-[(2R)-2-(2-Sulfanylacetylamino)-3-sulfopropyl]-3-sulfopropyl}ethyl)carbamoyl]propoxy}-2,6-dimethylphenyl)sulfonyl]amino}-(2S)-3-({7-[(imidazol-2-ylamino)methyl]-1-mehyl-4-oxo(3-hydroquinolyl)}carbonylamino)-propanoic Acid

A solution of the product of Part A, above (19.0 mg, 0.0122 mmol) in TFA (5.0 mL) was treated with triethylsilane (0.050 mL) and heated at 70 °C under nitrogen for 1 h. The solution was concentrated and dried under high vacuum. The resulting residue was purified by HPLC on a Phenomenex Luna C-18 column (21.2 x 250 mm) using a 0.675%/min gradient of 0 to 27% ACN containing 0.1% TFA at a flow rate of 20 mL/min. The main product peak eluting at 29 min was lyophilized to give the title compound as a colorless solid (6.6 mg, 50.4%). MS: m/e 1073.6 [M+H] (100%), 537.3 [M+2H] (90%); High Resolution MS: Calcd for C₄₀H₅₀N₁₀O₁₇S₄ [M-2H]: 535.1119; Found: 535.1116.

### Example 5

### Synthesis of Receptor-Targeted Ultrasound Contrast Agent

### Part A - Preparation of Maleimide-Functionalized Microbubbles

1,2-Dioleoyl-sn-glycero-3-phosphoethanolamine-N-[4-(p-maleimidomethyl)-cyclohexanecarboxamide] is admixed with three other lipids, 1,2-dipalmitoyl-sn-glycero-3-phosphotidic acid, 1,2-dipalmitoyl-sn-glycero-3-phosphatidylcholine, and N-(methoxypolyethylene glycol 5000 carbamoyl)-1,2-dipalmitoyl-sn-glycero-3-phosphatidylethanolamine in relative amounts of 1 wt.% : 6 wt.% : 54 wt.% : 41 wt.%. An aqueous solution of this lipid admixture (1 mg/mL), sodium chloride (7 mg/mL), glycerin (0.1 mL/mL), and propylene glycol (0.1 mL/mL) at pH 6-7 is then prepared in a 2 cc glass vial. The air in the vial is evacuated and replaced with perfluoropropane and the vial is sealed. The microbubbles are formed by agitating the sealed vial in a dental amalgamator for 30-45 seconds to form a milky white solution.

### Part B - Conjugation of 2-{[(4-{3-[N-(2-{(2R)-2-[(2R)-2-(2-Sulfanylacetylamino)-3-sulfopropyl]-3-sulfopropyl}ethyl)carbamoyl]propoxy}-2,6-dimethylphenyl)sulfonyl]amino}-(2S)-3-({7-[(imidazol-2-ylamino)methyl]-1-methyl-4-oxo(3-hydroquinolyl)}carbonylamino)-propanoic Acid to Maleimide-Functionalized Ultrasound Contrast Agent

The microbubble suspension that is prepared in Part A above is treated with the product of Example 4 and mixed gently at 0 °C until HPLC analysis shows completion of the conjugation reaction. An aqueous solution of ethanethiol is added to block unreacted maleimide groups. The microbubbles are purified by gentle centrifugation. The ultrasound contrast agent composition is completed by resuspending the bubbles in phosphate buffer.

The disclosures of each patent, patent application, and publication cited or described in this document are hereby incorporated herein by reference, in their entireties.

Various modifications of the invention, in addition to those described herein, will be apparent to those skilled in the art from the foregoing description. Such modifications are also intended to fall within the scope of the appended claims.

## Claims

1. Compound of the formula (V): 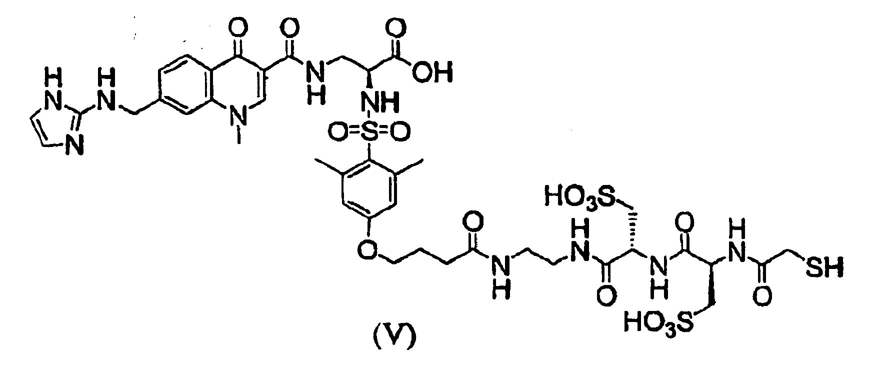 or a pharmaceutically acceptable salt thereof.

2. A method of preparing the compound of claim 1, comprising: contacting a compound of the formula (VIa): 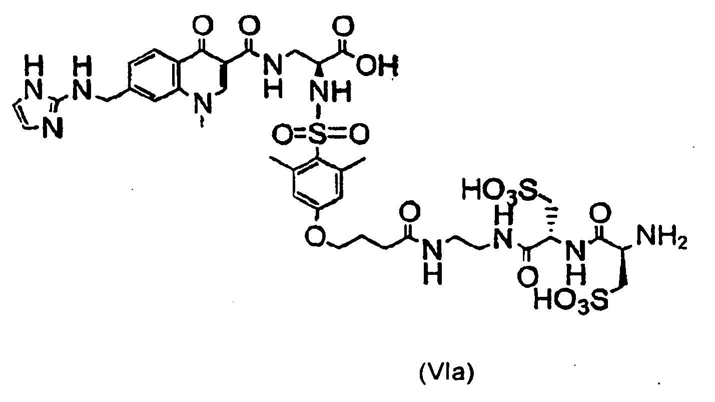 which may further comprise one or more protecting group(s), with a compound having the formula (VIIa): 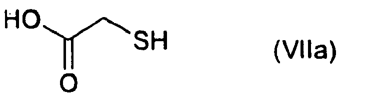 which may further comprise one or more protecting group(s);
optionally removing the protecting group(s); and
recovering the compound of claim 1.

3. A compound, comprising a thiol derivatized targeting moiety of the formula (V): 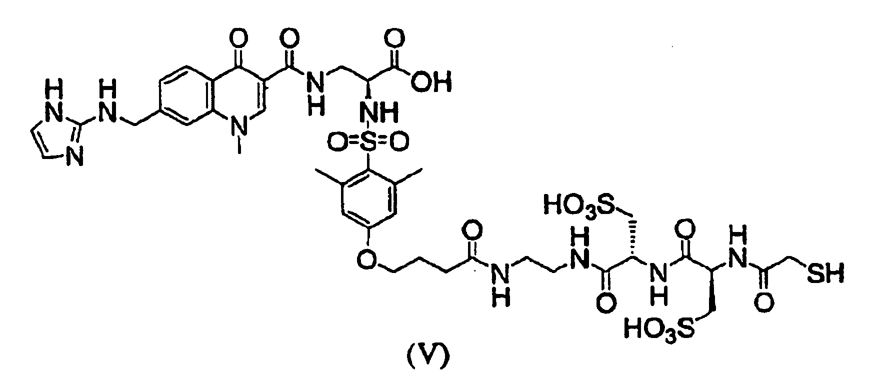 or a pharmaceutically acceptable salt thereof.

4. A method of preparing a contrast agent, comprising:
contacting the compound of claim 1 or a pharmaceutically acceptable salt thereof with a maleimide derivatized imageable moiety.

5. A method of preparing a contrast agent, comprising:
contacting the compound of claim 1 or a pharmaceutically acceptable salt thereof with a α-haloacetyl derivatized imageable moiety.

## Patentansprüche

1. Verbindung der Formel (V): 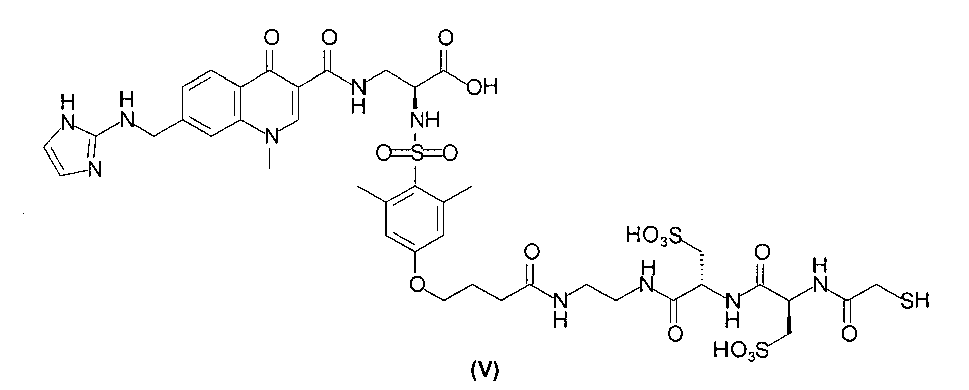 oder ein pharmazeutisch verträgliches Salz davon.

2. Verfahren zur Herstellung der Verbindung von Anspruch 1, umfassend: das Inkontaktbringen einer Verbindung der Formel (VIa): 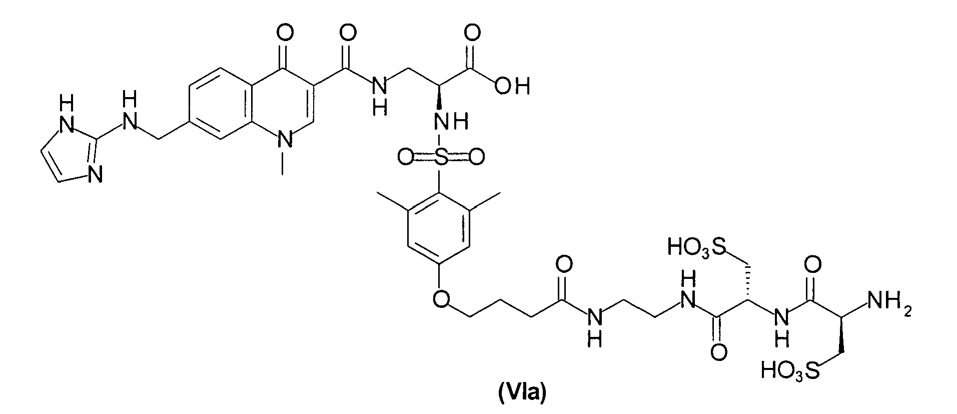 die zusätzlich eine oder mehrere Schutzgruppe(n) umfassen kann, mit einer Verbindung mit der Formel (VIIa): 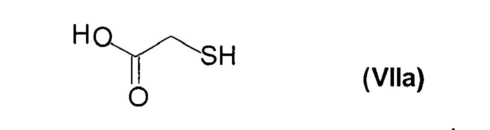 die zusätzlich eine oder mehrere Schutzgruppe(n) umfassen kann;
das optionale Entfernen der Schutzgruppe(n) und
die Rückgewinnung der Verbindung von Anspruch 1.

3. Verbindung, umfassend eine Thiol-derivatisierte Targeting-Komponente der Formel (V): 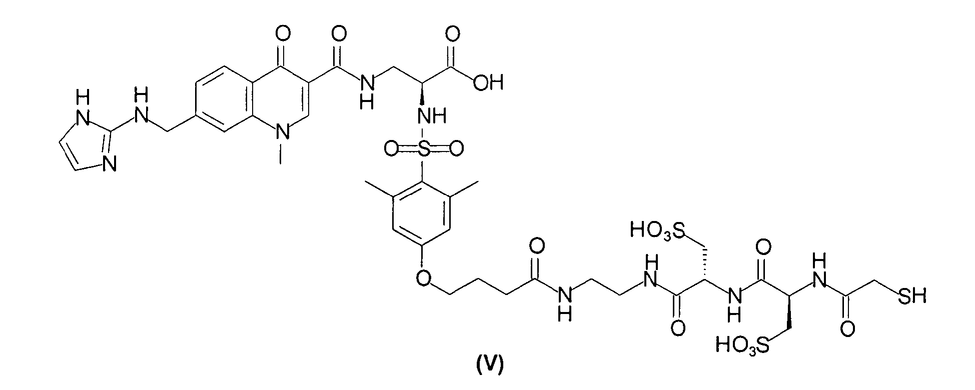 oder ein pharmazeutisch verträgliches Salz davon.

4. Verfahren zur Herstellung eines Kontrastmittels, umfassend:
das Inkontaktbringen der Verbindung von Anspruch 1 oder eines pharmazeutisch verträglichen Salzes davon mit einer Maleimid-derivatisierten abbildbaren Komponente.

5. Verfahren zur Herstellung eines Kontrastmittels, umfassend:
das Inkontaktbringen der Verbindung von Anspruch 1 oder eines pharmazeutisch verträglichen Salzes davon mit einer α-Haloacetylderivatisierten abbildbaren Komponente.

## Revendications

1. Composé de formule (V) : 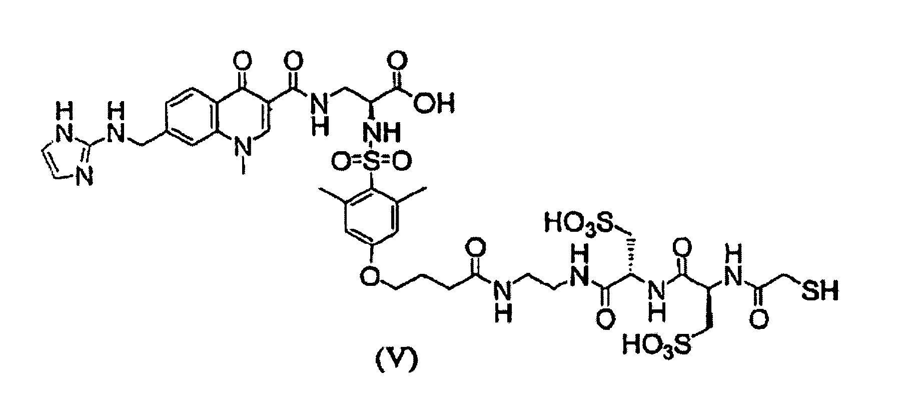 ou un sel pharmaceutiquement acceptable de celui-ci.

2. Procédé de préparation du composé selon la revendication 1, comprenant :
la mise en contact d'un composé de formule (VIa) : 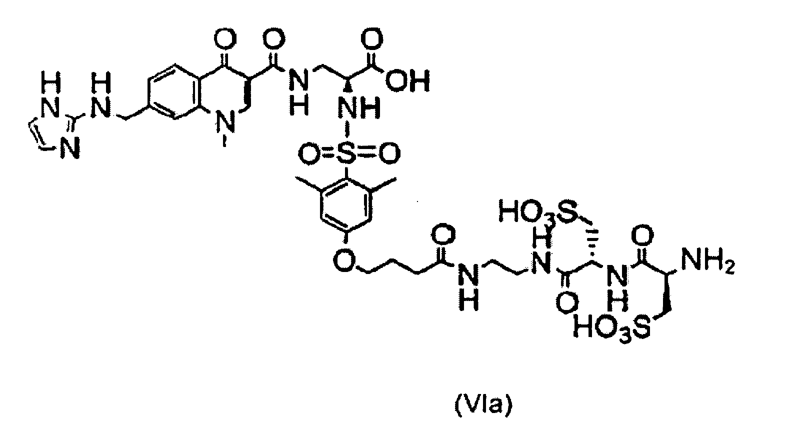
qui peut comprendre en outre un ou plusieurs groupe(s) protecteur(s),
avec un composé ayant la formule (VIIa) : 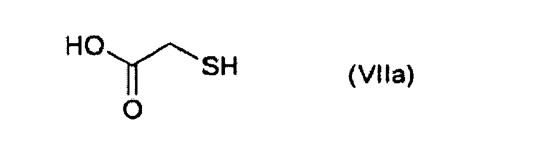
qui peut comprendre en outre un ou plusieurs groupe(s) protecteur(s) ;
éventuellement l'élimination du/des groupe(s) protecteur(s) ; et
la récupération du composé selon la revendication 1.

3. Composé comprenant un radical de ciblage dérivatisé par un thiol de formule (V) : 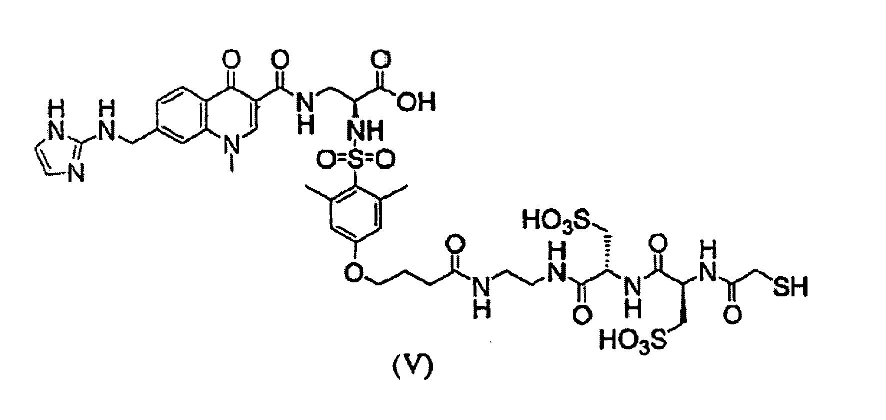 ou un sel pharmaceutiquement acceptable de celui-ci.

4. Procédé de préparation d'un agent de contraste, comprenant :
la mise en contact du composé selon la revendication 1 ou d'un sel pharmaceutiquement acceptable de celui-ci avec un radical imageable dérivatisé par un maléimide.

5. Procédé de préparation d'un agent de contraste, comprenant :
la mise en contact du composé selon la revendication 1 ou d'un sel pharmaceutiquement acceptable de celui-ci avec un radical imageable dérivatisé par un α-halogénoacétyle.
